# EUROPEAN PATENT SPECIFICATION

(11) **EP 3 381 458 B1**
(45) Date of publication and mention of the grant of the patent: **07.06.2023**
(21) Application number: 16868457.9
(22) Date of filing: 17.11.2016
(51) Int. Cl.: A61K 35/28, A61P 1/16

(54) **THERAPEUTIC AGENT FOR LIVER DISEASE CONTAINING STROMAL CELLS DERIVED FROM ADIPOSE TISSUE AND METHOD FOR PRODUCING THE SAME**
THERAPEUTISCHES MITTEL FÜR LEBERERKRANKUNGEN MIT STROMAZELLEN AUS FETTGEWEBE UND VERFAHREN ZUR HERSTELLUNG DIESES THERAPEUTISCHEN MITTELS
AGENT THÉRAPEUTIQUE POUR MALADIE HÉPATIQUE COMPRENANT DES CELLULES STROMALES DÉRIVÉES DE TISSU ADIPEUX, ET PROCÉDÉ DE PRODUCTION DUDIT AGENT THÉRAPEUTIQUE

(30) Priority: 24.11.2015 JP 2015228731
(43) Date of publication of application: 03.10.2018
(73) Proprietor: Rohto Pharmaceutical Co., Ltd., Osaka-shi Osaka 544-8666 (JP)
(72) Inventor: KURATA, Hayato, Osaka-shi Osaka 544-8666 (JP); NONAKA, Hidenori, Osaka-shi Osaka 544-8666 (JP)
(74) Representative: Mewburn Ellis LLP
(86) International application number: PCT/JP2016/084127
(87) International publication number: WO 2017/090509

(56) References cited:
- EP-A1- 2 554 176
- WO-A1-2008/140141
- WO-A1-2016/001839
- WO-A2-2009/115581
- JP-A- 2009 001 509
- JP-A- 2012 056 913
- JP-A- 2012 149 088
- JP-A- 2012 533 620
- JP-A- 2013 528 368
- JP-A- 2014 527 821
- JP-A- 2015 529 687
- JP-A- 2016 138 092
- KATSUDA TAKESHI ET AL: "The In Vivo Evaluation of the Therapeutic Potential of Human Adipose Tissue-Derived Mesenchymal Stem Cells for Acute Liver Disease", ANIMAL MODELS FOR STEM CELL THERAPY HUMANA PRESS INC, 999 RIVERVIEW DR, STE 208, TOTOWA, NJ 07512-1165 USA SERIES : METHODS IN MOLECULAR BIOLOGY (ISSN 1064-3745(PRINT)), 2014, pages 57-67, XP009513759,
- MINONZIO GRETA ET AL: "Frozen adipose-derived mesenchymal stem cells maintain high capability to grow and differentiate", CRYOBIOLOGY, ACADEMIC PRESS INC, US, vol. 69, no. 2, 15 July 2014 (2014-07-15), pages 211-216, XP029071727, ISSN: 0011-2240, DOI: 10.1016/J.CRYOBIOL.2014.07.005
- EIHEI TO et al.: "Shibo Yurai Kan'yo-kei Kansaibo Ishoku ni yoru Kankohen Kaizen Koka ni Tsuite no Jikkenteki no Kento", Regenerative Medicine, vol. 14, February 2015 (2015-02), page 329, XP9511394,
- YOSHIO SAKAI et al.: "Saisei Iryo no Rinsho Kenkyu·Chiken Kankohen ni Taisuru Jiko Shibo Soshiki Yurai Kanshitsu Saibo no Kei Kandomyaku Toyo ni yoru Kan Saisei Ryoho", Japanese Journal of Clinical Medicine, vol. 73, no. 5, June 2015 (2015-06), pages 492-498, XP009510580,
- SUMIDA,S et al.: "The repetitive freeze-thaw- culture method (RFTCM) to preserve human adipose tissue (-33years at -196°c) and the adipocyte derived mesenchymal stromal cells (MSCs) for regenerative medicine.", Low Temperature Medicine, vol. 41, no. 1, October 2015 (2015-10), page 49,
- MAMIDI, MK et al.: "Comparative Cellular and Molecular Analyses of Pooled Bone Marrow Multipotent Mesenchymal Stromal Cells During Continuous Passaging and After Successive Cryopreservation", J. Cell Biochem., vol. 113, no. 10, 2012, pages 3153-3164, XP055182430,
- HAYATO KURATA et al.: "Hito Shibo Soshiki Yurai Kan'yo-kei Kansaibo wa Exosomes Bunpitsu o Kaishite Kanshogai o Yokusei suru", Regenerative Medicine, vol. 15, February 2016 (2016-02), XP9511393,
- LIU G ET AL: "Evaluation of the viability and osteogenic differentiation of cryopreserved human adipose-derived stem cells", CRYOBIOLOGY, ACADEMIC PRESS INC, US, vol. 57, no. 1, 1 August 2008 (2008-08-01) , pages 18-24, XP023314992, ISSN: 0011-2240, DOI: 10.1016/J.CRYOBIOL.2008.04.002 [retrieved on 2008-05-21]

## Description

### TECHNICAL FIELD

The present invention relates to a therapeutic agent for liver diseases containing stromal cells derived from adipose tissue and a method for producing the same.

### BACKGROUND ART

Stromal stem cells are somatic stem cells that have been pointed out to be present in the bone marrow, and have an ability to differentiate into bone, cartilage and fat as stem cells, and therefore are attracting attention as a promising cell source in cell therapy. Recently, it has been known that there exist cells having equivalent functions in stromal cells derived from adipose tissue and fetal appendages such as placenta, umbilical cord, egg membrane, etc. For this reason, the stromalcells are sometimes called as mesenchymal stem cells.

The stromal cells are also attracting attention because they have various functions other than their differentiation ability, and using the stromal cells derived from bone marrow, tests are progressing on acute graft-versus-host disease (GVHD) in hematopoietic stem cell transplantation, Crohn disease of inflammatory bowel disease, acute myocardial infarction, and ischemic heart failure.

Adipose tissue can give many stromal cells at one time operation as compared with bone marrow and the degree of invasion upon collecting from the donor is also lower than that of bone marrow, so that stromal cells derived from adipose tissue are attracting attention as a therapeutic agent for cell transplantation (Patent documents 1 and 2). However, stromal cells derived from bone marrow and stromal cells derived from adipose tissue do not necessarily exhibit the same properties, and differences in their growth rate and differentiation trends have also been reported (Non-Patent document 1).

Until now, it has been suggested to use the stromal cells derived from adipose tissue for treatment of liver diseases, in particular, for treatment of liver cirrhosis (Non-Patent documents 1 to 4), but the therapeutic effect, or the producing method and the preservation method of the stromal cells derived from adipose tissue for heightening convenience at the time of the treatment has not been disclosed.

Studies to examine the therapeutic effect of liver cirrhosis using stromal cells derived from adipose tissue are planned in Japan, but these are all autologous (syngeneic) cell transplantation. However, for carrying out cell transplantation therapy in a universal manner, it is desirable to carry out the allotransplantation (allogeneic) in the future, but, in particular, studies considering the therapeutic effect on liver diseases, and improvement of convenience at the time of the treatment are not sufficiently carried out with regard to the producing method and the preservation method of the stromal cells derived from adipose tissue for allotransplantation.

In addition, there are reports that the cells subjected to cryopreservation are attenuated in the therapeutic effect of the cells or side effects are increased as compared with the cells not subjected to cryopreservation, etc. (Non-Patent documents 5 to 7), so that it is thought that it is desirable not to carry out cryopreservation when the cells are used for the treatment.

Katsuda T., et al., Methods in Molecular Biology, 1213: 57-66, 2014, discloses a method of evaluating the therapeutic efficacy of systemic administration of adipose tissue-derived mesenchymal stem cells in mouse models of acute hepatitis.

EP 2 554 176 A1 discloses compositions comprising human adipose stem cells for the prevention, amelioration or treatment of acute or chronic liver disease.

WO 2008/140141 discloses pharmaceutical compositions comprising mesenchymal stem cells for preventing and treating liver fibrosis and hepatic cirrhosis.

Mamidi, MK., et al., J. Cell. Biochem. 113(10): 3153-3164, 2012, discloses successive or multiple rounds of cryopreservation of mesenchymal stem cells does not alter the fundamental characteristics of mesenchymal stem cells, such as differentiation potential, growth and gene expression patterns.

### PRIOR ART DOCUMENTS

### PATENT DOCUMENTS

Patent document 1: U.S. Patent No. 6,777,231
Patent document 2: JP 2013-13411A

### NON-PATENT DOCUMENT

Non-Patent document 1: Yu F., et. al., Journal of the Formosan Medical Association, 114, 130-138, 2015
Non-Patent document 2: Banas A., et. al., Stem Cells. 26: 2705-2712, 2008
Non-Patent document 3: Harn H., et. al., Cell Transplant., 21: 2753-2764, 2012
Non-Patent document 4: Salomone F., et. al., Stem Cell Res., 11:1037-1044, 2013
Non-Patent document 5: Francois M., et. al., Cytotherapy, 14: 147-152, 2012
Non-Patent document 6: Moll G., et. al., Stem Cells, 32(9): 2430-2442, 2014
Non-Patent document 7: Quimby J. M., et. al., Stem Cell Res. Ther., 4(2): 48-59, 2013

### SUMMARY OF THE INVENTION

### PROBLEMS TO BE SOLVED BY THE INVENTION

When cryopreserved cells are used, after thawing, it is difficult to predict whether cells have the same function as before freezing or not (Non-Patent documents 5 to 7). In particular, when stromal cells derived from adipose tissue are used for the treatment of liver diseases, since the mechanism of the function of the cells to be effective for treating liver diseases is unknown, it is particularly difficult to estimate the effectiveness of the cells after freezing and thawing on the treatment of liver diseases. In addition, when the stromal cells derived from adipose tissue subjected to freezing and thawing repeatedly are administered, it cannot be presumed that a therapeutic effect on liver diseases will occur.

Further, when it is necessary to culture stromal cells derived from adipose tissue immediately before administration, the medical institution needs to prepare a special culture facility, so that there is a fear of lowering versatility. Thus, in order to improve convenience, it is desirable to develop a method for preparing preserved stromal cells derived from adipose tissue for transplantation as they are. For preservation of the cells, it is necessary to freeze the cells at low temperature, but the preservation solution used at the time of such freezing has been developed for the purpose of maintaining cell function such as cell karyotype, proliferative ability, differentiation ability, etc., and of improving cell viability, and it has not been developed on the assumption that the cells are administered to the body together with the preservation solution. In addition, when the cryopreservation solution is to be evaluated, it is evaluated after activating cells through the culturing step, so that, after thawing, it is unknown that the cells that are to be not cultured is in what kind of situation, and there is no example focusing on such a point. Indeed, it is unknown whether or not it is effective for the treatment of liver diseases to prepare the cells after thawing as they are and administer the same.

The present invention has been made in view of the above problems, and objects thereof are to provide a method for producing a therapeutic agent for liver diseases containing stromal cells derived from adipose tissue which is excellent in convenience of preparation of a therapeutic agent while maintaining an excellent therapeutic effect on liver diseases of the stromal cells derived from adipose tissue, and a therapeutic agent for liver diseases obtained by such a producing method.

### MEANS TO SOLVE THE PROBLEMS

That is, the present invention relates to the following:
[1] A therapeutic agent for use in treatment of liver diseases, which comprises stromal cells derived from adipose tissue that have been subjected to both cryopreservation and thawing at least twice, wherein the treatment comprises administering the cells immediately after said thawing.
[2] The therapeutic agent for use in treatment of liver diseases described in [1], which is for treatment of hepatitis.
[3] The therapeutic agent for use in treatment of liver diseases described in [1], which is for treatment of liver cirrhosis.
[4] The therapeutic agent for use in treatment of liver diseases described in any one of [1] to [3], which is for suppressing liver fibrosis.
[5] The therapeutic agent for use in treatment of liver diseases described in any one of [1] to [4], the stromal cells derived from adipose tissue are allogeneic to a subject.
[6] The therapeutic agent for use in treatment of liver diseases described in any one of [1] to [5], the stromal cells derived from adipose tissue are human stromal cells derived from adipose tissue.
[7] The therapeutic agent for use in treatment of liver diseases described in any one of [1] to [6], wherein an action of subjecting to both cryopreservation and thawing at least twice contains the following steps of;
   (1) a step of cryopreserving and thawing stromal cells derived from adipose tissue,
   (2) a step of culturing the thawed cells, and
   (3) a step of cryopreserving and thawing the cultured cells.
[8] The therapeutic agent for use in treatment of liver diseases described in any one of [1] to [7], wherein the stromal cells derived from adipose tissue are stromal cells derived from adipose tissue at passage 4 or less.
[9] The therapeutic agent for use in treatment of liver diseases described in any one of [1] to [8], wherein the cryopreservation is carried out in a gas phase on a liquid nitrogen.
[10] The therapeutic agent for use in treatment of liver diseases described in any one of [1] to [9], wherein the cryopreservation is carried out by using a solution containing DMSO (Dimethyl sulfoxide).
[11] A method for producing a therapeutic agent for use in treatment of liver diseases containing stromal cells derived from adipose tissue which comprises
   (i) a step of culturing stromal cells separated from collected adipose tissue,
   (ii) a step of subjecting to both cryopreservation and thawing the stromal cells derived from adipose tissue obtained in the step (i),
   (iii) a step of subjecting to both cryopreservation and thawing the thawed stromal cells derived from adipose tissue, and
   (iv) a step of mixing the thawed stromal cells derived from adipose tissue in the step (iii) with an infusion preparation.
[12] The producing method described in [11], wherein the cryopreservation is carried out by using a cryopreservation solution, and the step (iv) is a step of mixing a cryopreservation solution containing the thawed stromal cells derived from adipose tissue with an infusion preparation.
[13] The producing method described in [11] or [12], wherein the stromal cells derived from adipose tissue are human stromal cells derived from adipose tissue.
[14] The producing method described in any one of [11] to [13], wherein the culturing in the step (i) is carried out at least one passage.
[15] The producing method described in any one of [11] to [14], wherein a step of culturing the thawed stromal cells derived from adipose tissue is further contained in the step (ii).
[16] The producing method described in any one of [11] to [15], wherein the cryopreservation is carried out in a vapor phase on a liquid nitrogen.
[17] The producing method described in any one of [12] to [16], wherein the cryopreservation solution is a solution containing DMSO.

### EFFECTS OF THE INVENTION

Accordingly, the present invention is to provide a therapeutic agent for use in treatment of liver diseases containing the cryopreserved and thawed stromal cells derived from adipose tissue. The present invention is further to provide a method for producing a therapeutic agent for use in treatment of liver diseases containing cryopreserved stromal cells derived from adipose tissue.

### BRIEF DESCRIPTION OF THE DRAWINGS

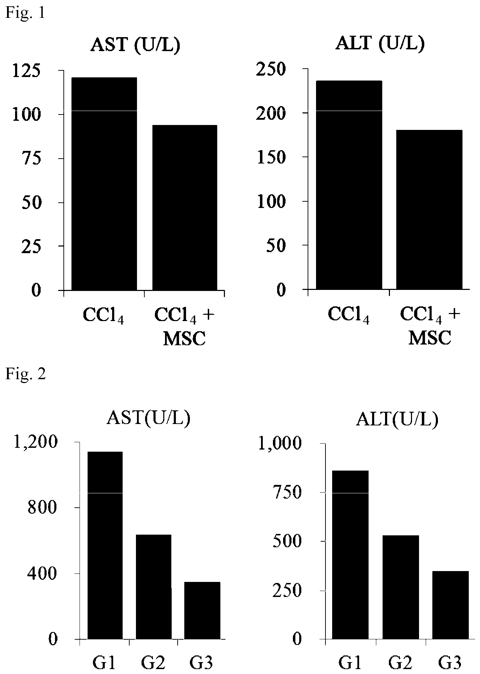

Fig. 1 shows the results of measurement of AST (Aspartate transaminase) (left figure) and ALT (Alanine transaminase) (right figure) in serum after administration of human stromal cells derived from adipose tissue to liver cirrhosis model mice prepared by administering 0.5 mL/kg of carbon tetrachloride (CCl₄). In the figure, CCl₄ shows a control group in which a Ringer's lactate solution alone was administered, and CCl₄+MSC shows the treatment group in which human stromal cells derived from adipose tissue were administered.
Fig. 2 shows the results of measurement of AST (left figure) and ALT (right figure) in serum after administration of human stromal cells derived from adipose tissue to acute hepatitis model mice prepared by administering 0.0625 mL/kg of CCl₄. In the figure, G1 is the result of the control group in which PBS alone was administered, G2 is the result of the treatment group in which human stromal cells derived from adipose tissue after culturing were administered, and G3 is the result of the treatment group in which human stromal cells derived from adipose tissue immediately after thawing of freezing were administered.

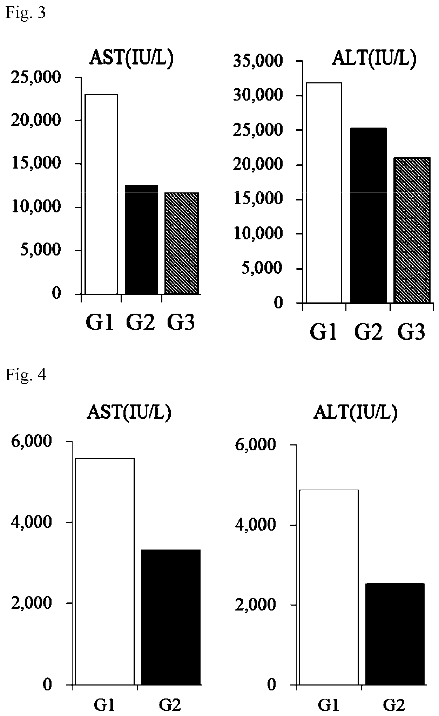

Fig. 3 shows the results of measurement of AST (left figure) and ALT (right figure) in serum after administration of human stromal cells derived from adipose tissue to before and after administration of CCl₄ of acute hepatitis model mice prepared by administering 0.0625 mL/kg of CCl₄. In the figure, G1 is the result of the control group in which PBS alone was administered, G2 is the result of the treatment group in which human stromal cells derived from adipose tissue was administered before administering CCl₄, and G3 is the result of the treatment group in which human stromal cells derived from adipose tissue was administered after administering CCl₄.
Fig. 4 shows the results of measurement of AST (left panel) and ALT (right panel) in serum after administering of human stromal cells derived from adipose tissue to fulminant hepatitis model mice prepared by administering 20 mL/kg of concanavalin A type IV, In the figure, G1 is the result of a control group in which PBS alone was administered and G2 is the result of the treatment group in which human stromal cells derived from were administered.

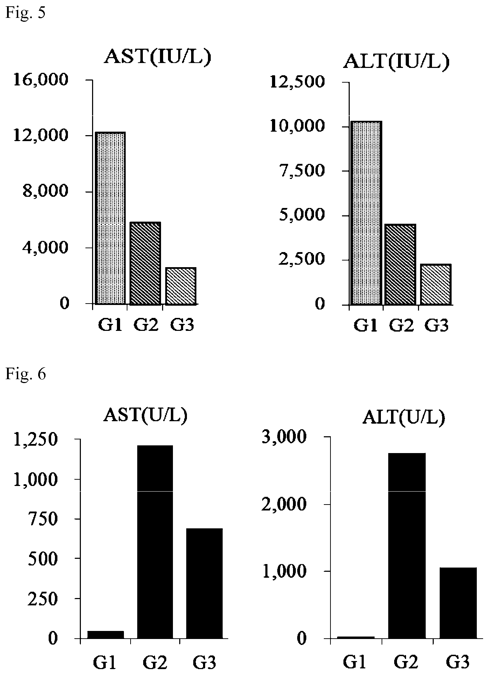

Fig. 5 shows the results of measurement of AST (left figure) and ALT (right figure) in serum after administration of each dose of human stromal cells derived from adipose tissue to fulminant hepatitis model mice prepared by administering 20 mL/kg of concanavalin A type IV. In the figure, G1 is the result of the control group in which a Ringer's lactate solution alone was administered, G2 is the result of the treatment group in which human stromal cells derived from adipose tissue were administered with a medium dose (1.5×10⁷ cells/kg), and G3 is the result of the treatment group in which human stromal cells derived from adipose tissue were administered with a high dose (5×10⁷ cells/kg).
Fig. 6 shows the results of measurement of AST (left figure) and ALT (right figure) in serum after administration of STEM-CELLBANKER to healthy mice or after administration of STEM-CELLBANKER or human stromal cells derived from adipose tissue dissolved in STEM-CELLBANKER to acute hepatitis model mice prepared by administering 0.0625 mL/kg of CCl₄. In the figure, G1 is the result of the control group in which STEM-CELLBANKER alone was administered to healthy mice, G2 is the result of the control group in which STEM-CELLBANKER was administered to acute hepatitis model mice, and G3 is the result of the treatment group in which human stromal cells derived from adipose tissue suspended in STEM-CELLBANKER were administered to acute hepatitis model mice.

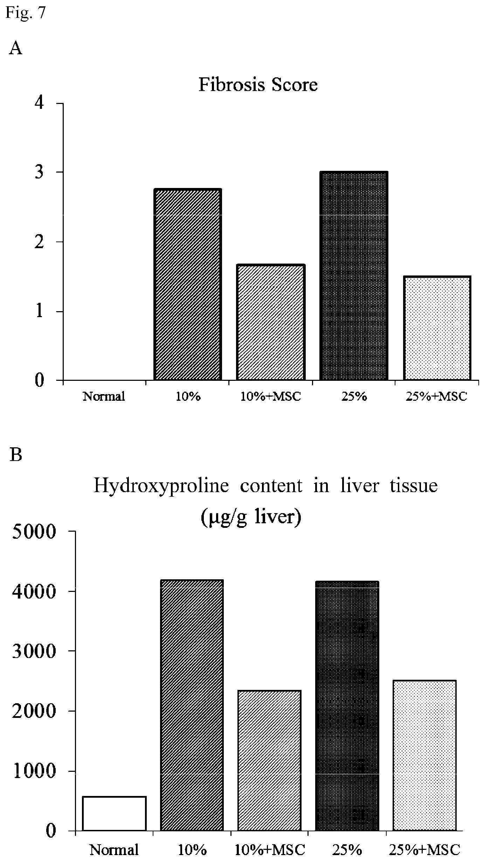

Fig. 7A shows the result of fibrosis score from Masson's trichrome staining of isolated liver after administration of human stromal cells derived from adipose tissue to liver cirrhosis model mice prepared by administering 1 mL/kg of each concentration of CCl₄. Fig. 7B shows the result of measurement of an amount of hydroxyproline in isolated liver after administration of human stromal cells derived from adipose tissue to liver cirrhosis model mice prepared by administering 1 mL/kg of each concentration of CCl₄. In the figure, Normal is the result of healthy mice, 10% is the result of liver cirrhosis model mice prepared by administering of 10v/v% CCl₄, 10%+MSC is the result of the treatment group in which human stromal cells derived from adipose tissue were administered to liver cirrhosis model mice prepared by administering of 10v/v%CCl₄, 25% is the result of liver cirrhosis model mice prepared by administering of 25v/v%CCl₄, and 25%+MSC is the result of the treatment group in which human stromal cells derived from adipose tissue were administered to liver cirrhosis model mice prepared by administering of 25v/v%CCl₄.

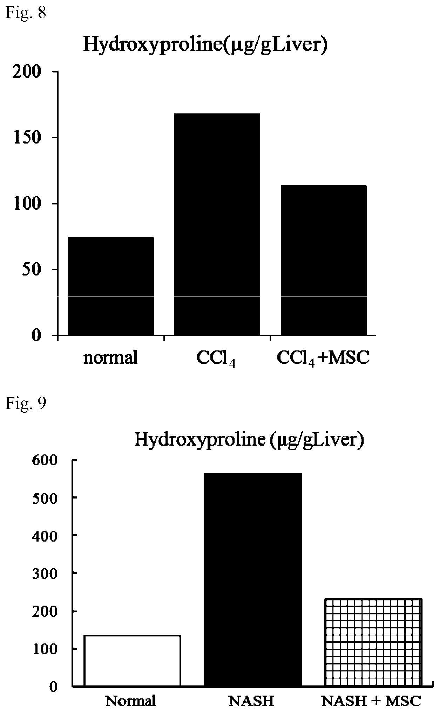

Fig. 8 shows the results of measurement of an amount of hydroxyproline in isolated liver after administration of human stromal cells derived from adipose tissue to liver cirrhosis model mice prepared by multiple administration of CCl₄. In the figure, Normal is the results of healthy mice, CCl₄ is the results of the control group in which no human stromal cells derived from adipose tissue were administered to liver cirrhosis model mice, and CCl₄+MSC is the results of the treatment group in which human stromal cells derived from adipose tissue were administered to liver cirrhosis model mice.
Fig. 9 shows the results of measurement of an amount of hydroxyproline in isolated liver after administration of human stromal cells derived from adipose tissue to non-alcoholic steatohepatitis model mice by feeding choline deficient amino acid-regulated diet. In the figure, Normal 1 is the results of healthy mice, NASH is the results of the control group in which no human stromal cells derived from adipose tissue was administered to non-alcoholic steatohepatitis model mice, and NASH+MSC is the results of the treatment group in which human stromal cells derived from adipose tissue were administered to non-alcoholic steatohepatitis model mice.

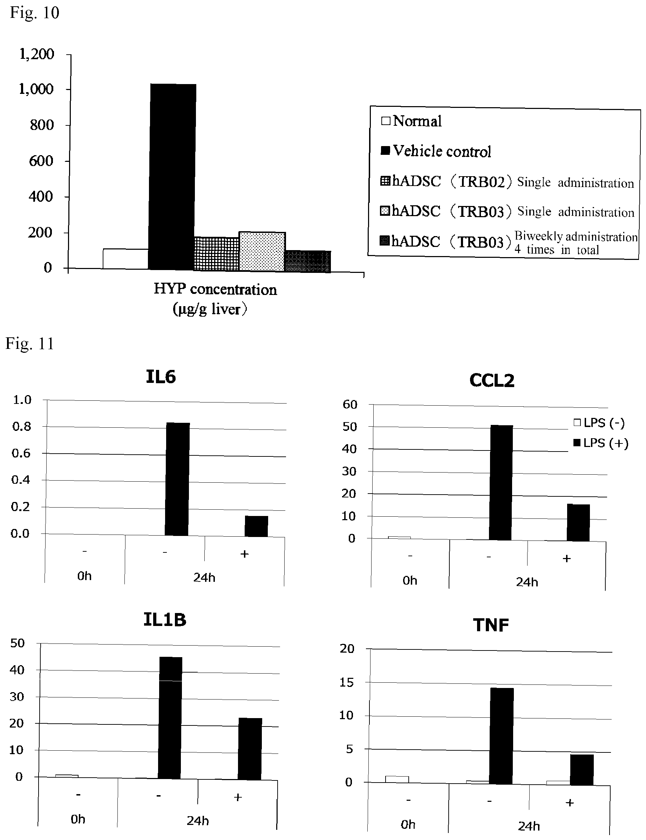

Fig. 10 shows the results of measurement of an amount of hydroxyproline in isolated liver after a single administration or multiple administration of human stromal cells derived from adipose tissue to liver cirrhosis model mice prepared by multiple administration of CCl₄. In the figure, Normal is the results of healthy mice, Vehicle control is the results of the control group in which no human stromal cells derived from adipose tissue was administered to liver cirrhosis model mice, hADSC (TRB02) single administration is the results of the treatment group in which stromal cells derived from adipose tissue (TRB02) were single administered to liver cirrhosis model mice, hADSC (TRB03) single administration is the results of the treatment group in which human stromal cells derived from adipose tissue (TRB03) were single administered to liver cirrhosis model mice, and hADSC (TRB03) biweekly 4 times administration in total is the results of the treatment group in which human stromal cells derived from adipose tissue (TRB03) were multiple administered to liver cirrhosis model mice.
Fig. 11 shows the results of quantitative PCR measurement of each inflammatory marker (IL6, CCL2, IL1β and TNF) in macrophages when co-culturing macrophages derived from THP1 cells and human stromal cells derived from adipose tissue. In the figure, the white bar is the results of no LPS stimulation, and the black bar is the results with LPS stimulation. "-" indicates macrophages derived from THP1 cells alone, and "+" indicates co-culturing of macrophages derived from THP1 cells and human stromal cells derived from adipose tissue.

### EMBODIMENTS TO CARRY OUT THE INVENTION

The invention is as defined in the appended claims.

In the present invention, the terms "adipose tissue" mean a tissue containing stromal cells which contain adipocytes and microvascular cells and, for example, a tissue obtained by surgical excision or suction of the subcutaneous adipose of a mammal. It is preferably subcutaneous adipose of human. Subcutaneous adipose may be obtained from a living or deceased individual, and the adipose tissue used in the present invention is preferably a tissue collected from a living individual. When it is collected from an individual, liposuction may be exemplified by, for example, PAL (power assist) liposuction, Elconia laser liposuction, or body jet liposuction and the like, and from the viewpoint of maintaining the state of the cell, it is preferable not to use ultrasonic waves, etc.

The "stromal cells" in the present invention mean cells which have an ability to differentiate into cells belonging to the mesenchymal system (osteocytes, cardiomyocytes, chondrocytes, tenocytes, adipocytes, etc.), and which can proliferate while maintaining the ability. In the present invention, the stromal cells mean the same cells as the stromal cells and are not particularly distinguishable. The stromal cells derived from adipose tissue mean stromal cells contained in the adipose tissue, and may be referred to as adipose derived stromal cells.

In the present invention, the stromal cells derived from adipose tissue mean any optional cell population containing stromal cells derived from adipose tissue. The cell population contains stromal cells derived from adipose tissue at least 20% or more thereof, preferably 30%, 40%, 50%, 60%, 70%, 75%, 80%, 85%, 90%, 93%, 96%, 97%, 98% or 99%.

The stromal cells derived from adipose tissue may be obtained by the producing method described in, for example, U.S. Patent No. 6,777,231, and produced by the method, for example, containing the following steps (a) to (c):
(a) a step of obtaining a cell suspension by digestion of adipose tissue with an enzyme;
(b) a step of precipitating the cells and resuspending the cells in an appropriate medium; and
(c) a step of culturing the cells on a solid surface and removing cells that do not show binding to the solid surface.

The adipose tissue used in step (a) is preferably a material which is washed. Washing can be carried out by using a physiologically compatible physiological saline solution (for example, phosphate buffered saline (PBS)), with vigorous stirring to sediment the tissue. This is to remove contaminants (also referred to as debris, such as damaged tissue, blood, erythrocytes, etc.) contained in the adipose tissue from the tissue. Accordingly, washing and sedimentation are generally repeated until the debris is totally eliminated from the supernatant. Since the remaining cells exist as clusters of various sizes, in order to dissociate while minimizing damage of the cells themselves, the mass of the cells after washing is preferably treated with an enzyme (for example, collagenase, Dispase, trypsin, etc.) that weakens or disrupts intercellular junctions. The amount of such enzyme and the duration of the treatment will vary depending on the conditions used, and are well known in the field of the art. Although the mass of the cells can be decomposed by other treatment methods such as mechanical stirring, ultrasonic energy, thermal energy, etc., in place of or in combination with such enzyme treatment, it is preferable to carry out it only with the enzyme treatment in order to minimize cell damage. When an enzyme is used, in order to minimize harmful effects to the cells, it is desirable to inactivate the enzyme using a medium, etc., after an appropriate period of time.

The cell suspension obtained by the step (a) contains a slurry or suspension of aggregated cells and various contaminating cells such as red blood cells, smooth muscle cells, endothelial cells, and fibroblasts. Accordingly, the cells in the aggregated state and these contaminating cells may be separated and removed, but since they can be removed by adhesion and washing in the step (c) mentioned later, the separation and removal may be omitted. When the contaminating cells are separated and removed, it can be achieved by centrifugation which compulsory separates the cells into supernatant and precipitate. The resulting precipitate containing contaminating cells is suspended in a physiologically compatible solvent. Whereas there is a fear that the cells in the suspended state contain the red blood cells, the red blood cells are excluded by selection by adhesion to the solid surface as mentioned later, so that the step of dissolving is not necessarily needed. As a method for selectively dissolving the red blood cells, well-known methods in the field of the art can be used, for example, incubation by dissolution with ammonium chloride in a hypertonic medium or a hypotonic medium, etc. After dissolution, the lysate may be separated from the desired cells by, for example, filtration, centrifugal sedimentation or density fractionation.

In the step (b), the suspended cells may be washed once or several times in succession, centrifuged and resuspended in a medium to increase purity of the stromal cells. Alternatively, the cells may be separated based on cell surface marker profiles or based on cell size and granularity.

The medium to be used for resuspension is not particularly limited as long as it is a medium capable of culturing stromal cells, and such a medium may be prepared by adding serum to the basal medium and/or adding one or more serum substitute, for example, albumin, transferrin, fatty acid, insulin, sodium selenite, collagen precursors, trace elements, 2-mercaptoethanol, 3'-thiol glycerol, etc. To these media may be further added, as necessary, substances such as lipids, amino acids, vitamins, growth factors, low molecular compounds, antibiotics, antioxidants, pyruvic acid, buffers, inorganic salts, etc. Such a medium is commercially available as a medium previously prepared for stromal cells (stromal cells) from, for example, PromoCell GmbH, Lonza, Biological Industries, Veritas, R&D Systems, Corning Inc., and ROHTO Pharmaceutical Co., Ltd. The basal medium may be mentioned, for example, IMDM medium, Medium 199 medium, Eagle's Minimum Essential Medium (EMEM) medium, αMEM medium, Dulbecco's modified Eagle's Medium (DMEM) medium, Ham's F12 medium, RPMI 1640 medium, Fischer's medium, and a mixed medium of these, etc. The serum may be mentioned, for example, human serum, fetal bovine serum (FBS), bovine serum, calf serum, goat serum, horse serum, swine serum, sheep serum, rabbit serum, rat serum, etc., and it is not limited by these. In the case of using serum, it may be added from 5 v/v% to 15 v/v%, preferably 10 v/v% based on the basal medium. The growth factors may be exemplified by, for example, platelet derived growth factor (PDGF), and basic fibroblast growth factor (bFGF), etc., but are not limited to these. The amino acids include, for example, L-alanine, L-arginine, L-aspartic acid, L-asparagine, L-cysteine, L-cystine, L-glutamic acid, L-glutamine, L-glycine, etc., but are not limited to these. From the viewpoint of using the stromal cells derived from adipose obtained in the present invention for cell transplantation, it is preferable to use a medium containing no heterologous components (xeno-free).

Subsequently, as the step (c), the cell suspension obtained in the step (b) is cultured on the solid surface without differentiation using the appropriate cell culture medium described above under appropriate cell density and culture conditions. In the present invention, the "solid surface" means any material that enables binding of the stromal cells derived from adipose tissue of the present invention. In a particular embodiment, such a material is a plastic material treated to promote the binding of mammalian cells to its surface. The shape of a culture vessel having a solid surface is not particularly limited, and a dish, a flask or the like is preferably used. To remove unbound cells and cell debris, cells are washed after incubation.

In the present invention, the cells that finally remain attached to the solid surface can be selected as a cell population of the stromal cells derived from adipose tissue.

Although it is not particularly limited, in order to confirm that the selected cells are the stromal cells derived from adipose tissue of the present invention, the surface antigen may be analyzed by a conventional method using flow cytometry or the like. In addition, the ability to differentiate into each cell line may be tested and such differentiation can be done by the conventional methods.

The stromal cells derived from adipose tissue of the present invention can be produced as mentioned above, and may be defined as cells having the characteristics comprising the following groups;
(a) under culture conditions in standard medium, it shows adhesiveness to plastics,
(b) the surface antigens CD105, CD73 and CD90 are positive, and CD45, CD34, CD11b, CD 19 and HLA-DR are negative, and
(c) it is possible to differentiate into osteocytes, adipocytes or chondrocytes under culture conditions.

The stromal cells derived from adipose tissue of the present invention may be repeatedly cryopreserved and thawed as appropriate for use as a therapeutic agent for liver diseases, preferably to carry out both cryopreservation and thawing at least twice and the number of times of the cryopreservation and thawing may be mentioned, for example, twice or more, three times or more, four times or more, and five times or more, and ten times or less, nine times or less, eight times or less, seven times or less, and six times or less. More preferably both cryopreservation and thawing are carried out twice. In the course of preparing a therapeutic agent for liver diseases containing the stromal cells derived from adipose tissue, by introducing the step of such both cryopreservation and thawing plural times, it is confirmed that the stromal cells are incompatible by the evaluation test, and when the cells are to be discarded, it is possible to suppress the production cost by avoiding the extra step(s) carried out after the thawing. In addition to the above, an operation can be interrupted during the process of preparing a therapeutic agent for liver diseases, whereby it becomes possible to artificial control of the producing step. Further, even if both cryopreservation and thawing are repeated twice or more as mentioned above, the therapeutic agent for liver diseases of the present invention retains excellent therapeutic effect. When such both cryopreservation and thawing are carried out at least twice, for example, a method including (1) a step of cryopreserving and thawing stromal cells derived from adipose tissue, (2) a step of culturing the thawed cells, and (3) a step of cryopreserving and thawing the cultured cells can be used. When adipose tissue collected from a donor is obtained, there may be mentioned a method containing (i) a step of culturing the stromal cells separated from the collected adipose tissue, (ii) a step of cryopreserving and thawing the stromal cells derived from adipose tissue obtained in the step (i), (iii) a step of cryopreserving and thawing the thawed stromal cells derived from adipose tissue, and (iv) a step of mixing the thawed stromal cells derived from adipose tissue in the step (iii) with an infusion preparation, preferably a method which further containing a step of culturing the thawed stromal cells derived from adipose tissue after the step (ii). If necessary, the step (iii) may be repeated. When the step (iii) is repeated, the method may further contain a step of culturing the stromal cells derived from adipose tissue prior to cryopreservation.

In the present invention, cryopreservation can be carried out by suspending the stromal cells derived from adipose tissue in the well known cryopreservation solution for the person skilled in the art and cooling the cells. The suspension can be carried out by scraping the cells with a release agent such as trypsin, transferring the cells into a cryopreservation container, treating them appropriately, and then adding a cryopreservation solution. The cryopreservation solution may be used a cryopreservation solution commercially available from, for example, Bio Verde Corporation, Japan Genetics Co., Reprocel, Xenoak, Cosmo Bio, Kojin Bio Co., Ltd., Thermo Fisher Scientific Co., etc.

Although the cryopreservation solution may contain DMSO (Dimethyl sulfoxide), DMSO has a property of inducing differentiation of stromal cells in addition to cytotoxicity, so it is preferable to reduce the content of DMSO. Glycerol or polysaccharide is exemplified as a substitute for DMSO. When DMSO is used, by containing it with a concentration of 5% to 20%, preferably a concentration of 5% to 10%, more preferably a concentration of 10%, it is possible to suppress cell damage at the time of freezing.

When the above-mentioned suspended cells are cryopreserved, it may be preserved at a temperature between -80°C and -100°C (for example, at -80°C), and an arbitrary freezer which can be attained at this temperature is used. Although it is not particularly limited, in order to avoid abrupt temperature change, the cooling rate may be appropriately controlled by using a program freezer. The cooling rate may be appropriately selected depending on the components of the cryopreservation solution and may be in accordance with the manufacturer's instruction of the cryopreservation solution.

The preservation period is not particularly limited and may be mentioned, for example, for 1 week or longer, 2 weeks or longer, 3 weeks or longer, 4 weeks or longer, 2 months or longer, 3 months or longer, 4 months or longer, 5 months or longer, 6 months or longer, one year or longer, or longer than these. Since it is possible to suppress cell damage by storing at a lower temperature, it may be preserved by transferring from the temperature between -80°C and -100°C to a gas phase on liquid nitrogen (about -150°C or lower to - 180°C or lower). When it is preserved in a gas phase on liquid nitrogen, it may be carried out by using a preservation container well known to those skilled in the art. Although it is not particularly limited, when it is preserved, for example, for 2 weeks or longer, it is preferable to preserve in gas phase on liquid nitrogen.

It is preferable to appropriately culture the thawed stromal cells derived from adipose tissue or the stromal cells separated from the adipose tissue before the next cryopreservation. Culturing is carried out using a medium capable of culturing the above-mentioned stromal cells, and is not particularly limited, it may be carried out at a culture temperature about 30 to 40°C, preferably about 37°C under an atmosphere of CO₂-containing air. The CO₂ concentration is about 2 to 5%, preferably about 5%. In the culture, passage may be carried out after reaching appropriate confluency (for example, there may be mentioned to occupy 50% to 80% of the area of the culture vessel by the cells) for the culture vessel. In the present invention, the passage means that cells are detached with a release agent such as trypsin and seeded in a separately prepared culture vessel at an appropriate cell density. In the passage, typical cell density may be exemplified by about 100 cells/cm² to about 100,000 cells/cm², about 500 cells/cm² to about 50,000 cells/cm², about 1,000 to 10,000 cells/cm², and about 2,000 to 10,000 cells/cm², etc. In a particular embodiment, the cell density is 2,000 to 10,000 cells/cm². It is preferable to adjust so that the period until reaching appropriate confluency is 3 days to 7 days. During the culture, if necessary, the medium may be appropriately exchanged.

Thawing of cryopreserved cells can be carried out by the method well known to those skilled in the art. For example, there may be exemplified by a method in which it is carried out by standing or shaking in a constant temperature bath at 37°C or in a hot water bath. The thawed cells be cultured before being cryopreserved again and may be prepared as a therapeutic agent to be mentioned later. When culturing is carried out, as in the above-mentioned passage, it can be carried out by seeding at an appropriate cell density in a separately prepared culture vessel.

The stromal cells derived from adipose tissue can be passaged several times without losing the therapeutic effect on liver diseases and can be increased in number depending on the culture method, and it is preferable that the number of passage times is smaller. On the other hand, in order to secure the number of cells necessary for treating liver diseases by culturing, it is preferable to carry out the passage. The stromal cells derived from adipose tissue used for the treatment of the present invention are preferably cells that have undergone the passage, for example, 8 times or less, 7 times or less, 6 times or less, 5 times or less, 4 times or less, or 3 times or less. More preferably, the cells that have undergone the passage 4 times or less. In the present invention, the operation in which cells may be detached, cryopreserved and thawed may be counted as one passage. As an embodiment to obtain such stromal cells derived from adipose tissue, there may be exemplified by (1) a step of culturing stromal cells separated from adipose tissue with at least one passage, (2) a step of cryopreservation, (3) a step of thawing, (4) a step of culturing, (5) a step of cryopreservation, and (6) a step of thawing.

The stromal cells derived from adipose tissue prepared above can be utilized as a therapeutic agent for liver diseases. In the present invention, the liver diseases are exemplified by hepatitis, liver cirrhosis, fatty liver, and the disease is made a classification by the cause such as viral, alcoholic, nonalcoholic, congenital, autoimmune, metabolic disorder and drug, a classification by the duration of disease such as acute and chronic, or a classification by the progress of the condition such as compensated and decompensated, and all of these classifications are included in the liver diseases. As presented in Example mentioned later, since fibrosis in the liver is suppressed by the stromal cells derived from adipose tissue, treatment of liver diseases also include suppression of hepatic fibrosis. In the present invention, the stromal cells derived from adipose tissue are suitably used for treatment of hepatitis, or liver cirrhosis.

For use as a therapeutic agent for liver diseases, the stromal cells derived from adipose tissue are desirably stromal cells isolated from human-derived adipose tissue, and from the viewpoint of using cryopreserved stromal cells derived from adipose tissue, liver diseases it is desirable to collect the adipose tissue from other subjects who are not subject to be administered suffered from liver disease. That is, the stromal cells derived from adipose tissue are preferably allogeneic to the subject.

According to the present invention, there is provided a therapeutic agent for liver diseases containing the above-mentioned stromal cells derived from adipose tissue as an active ingredient. Further, according to the present invention, there is provided the above-mentioned stromal cells derived from adipose tissue, which is used for cell transplantation therapy. Furthermore, in a non-claimed example, there are provided a method of transplanting cells to a subject and a method of treating a disease in a subject including a step of administering a therapeutically effective amount of the above-mentioned stromal cells derived from adipose tissue to a subject suffering from liver diseases,.

The cryopreserved stromal cells derived from adipose tissue obtained by the above-mentioned method are, after being thawed, mixed with an infusion preparation to produce a therapeutic agent for liver diseases including stromal cells derived from adipose tissue. In mixing, a cryopreservation solution in which cells after thawing are suspended may be mixed with an infusion preparation, or after separating the cells from a solvent by centrifugation, cells alone may be mixed with an infusion preparation. In order to avoid troublesome procedures, it is preferable not to include the step of culturing after thawing, or to mix the cryopreservation solution in which cells after thawing are suspended directly with an infusion preparation. This is as presented in Example 7 to be mentioned later, effectiveness of the procedure was confirmed by the fact that even when it is administered together with STEM-CELLBANKER commercially available from ZENOAQ Co., as a cryopreservation solution containing 10% DMSO, it is possible to reduce liver injury marker. The infusion preparation in which the cells are mixed may further contain a pharmaceutically acceptable carrier. In the present invention, the carrier may be exemplified by a diluent, an adjuvant, an excipient, a pH buffer, an isotonicity agent, a protein (for example, a serum component such as albumin, etc.) and a saccharide (for example, glucose) for administering a therapeutic agent. Incidentally, in a non-claimed example, the therapeutic agent for liver diseases is provided as a kit combining a frozen stromal cells derived from adipose tissue and an infusion preparation is also included.

The "infusion preparation" of the present invention is not particularly limited as long as it is a solution to be used for the treatment of human, and may be mentioned, for example, physiological saline, Japanese Pharmacopoeia physiological saline, 5% glucose solution, Japanese Pharmacopoeia glucose injection solution, Japanese Pharmacopoeia Ringer's solution, Ringer's lactate solution, Ringer's acetate solution, No. 1 solution (starting solution), No. 2 solution (dehydrated replenisher), No. 3 solution (maintaining solution), No. 4 solution (postoperative recovery solution), etc.

The method for administering the therapeutic agent for liver diseases is not particularly limited and may be mentioned, for example, subcutaneous injection, intralymphatic injection, intravenous injection, intraportal injection, intraperitoneal injection, intrapleural injection, intrahepatic arterial injection or direct injection into the liver or the spleen, etc., and preferably, intravenous injection, intraportal injection, intrasplenic administration.

The administration dose of the therapeutic agent for use in treatment of liver diseases of the present invention is a cell amount that can obtain a therapeutic effect on diseases when administered to a subject as compared with a subject not administered. The specific dose can be appropriately determined depending on the administration form, administration method, purpose of use, age, body weight and symptoms of the subject, etc., and as an example, a number of the stromal cells derived from adipose tissue is preferably 1×10⁵ to 1×10⁹ cells/kg body weight per one administration to human (for example, adult), more preferably 1×10⁵ to 1×10⁸ cells/kg body weight, and further preferably 1×10⁵ to 1×10⁷ cells/kg body weight.

The administration frequency of the therapeutic agent for use in treatment of liver diseases of the present invention may be once a day with the above administration dose, or may be administered by dividing into a plural times. Moreover, the above-mentioned dosage may be appropriately administered with an interval of a day(s). In the case of administration with an interval of a day(s), the dose at one time may be appropriately increased or decreased.

The therapeutic agent for use in treatment of liver diseases of the present invention may be used in combination with other therapeutic agents or may be used as a single agent.

### EXAMPLES

The present invention will be specifically explained with the following Examples, but the present invention is not limited by Examples.

### [Example 1] Preparation of therapeutic agent for liver diseases of the present invention

### Establishment of human stromal cells derived from adipose tissue

After obtaining consent from a human donor, subcutaneous adipose tissue obtained by the liposuction method was washed with physiological saline solution. In order to achieve destruction of extracellular matrix and isolation of cells, Liberase (Roche) (solvent is physiological saline) was added thereto, and shaken at 37°C for 90 minutes and dispersed. Subsequently, this suspension mentioned above was centrifuged at 800 g for 5 minutes to obtain a precipitate of the stromal vascular fraction.

To the above-mentioned precipitate of the cells was added a serum free medium for stromal cells (ROHTO PHARMACEUTICAL CO., LTD.), the cell suspension was centrifuged at 400 g for 5 minutes, and after removing the supernatant, the cells were resuspended in a serum free medium for stromal cells (ROHTO PHARMACEUTICAL CO., LTD.) and the cells were seeded in a flask.

The cells were cultured at 37°C for several days in a 5% CO₂ atmosphere. After several days, the cultures were washed with PBS to remove residual blood cells and adipose tissue contained in the cultures and stromal cells adhering to the container were obtained.

### Cryopreservation of human stromal cells derived from adipose tissue

The obtained human stromal cells derived from adipose tissue were apportioned into a centrifuge tube and centrifuged at 400 g for 5 minutes to obtain a precipitate of the cells. After removing the supernatant, a suitable amount of a cell cryopreservation solution (STEM-CELLBANKER (ZENOAQ Co.)) was added thereto to suspend the same. After the cell suspension solution was apportioned into cryotubes, these were preserved in a freezer at - 80°C, transferred to the gas phase on liquid nitrogen, and storage was continued.

### Thawing of human stromal cells derived from adipose tissue

The human stromal cells derived from adipose tissue cryopreserved in a cryotube was dissolved in a thermostatic chamber at 37°C, transferred into a centrifuge tube, diluted with a serum free medium for stromal cells (ROHTO PHARMACEUTICAL CO., LTD.), then, centrifuged at 400 g for 5 minutes to obtain a precipitate of the cells. After removing the supernatant, the cells were suspended in a serum free medium for stromal cells (ROHTO PHARMACEUTICAL CO., LTD.) and seeded in a flask. After several days, trypsin was added thereto, the cells were detached and diluted, and seeded in a new flask. An operation from seeding the treated subcutaneous adipose tissue to scraping by trypsin and recovery of the cells was made one passage, and it was carried out 4 passages in total to carry out expansion culture. The obtained human stromal cells derived from adipose tissue were cryopreserved as mentioned above. When it is administered to disease individuals, the frozen cells were thawed as mentioned in Example below, and a material suspended in a suitable solution was used as a therapeutic agent for liver diseases of the present invention.

### [Example 2] Investigation test 1 on therapeutic effect for liver cirrhosis

### Preparation of administration cells

The human stromal cells derived from adipose tissue preserved as mentioned above were thawed by the above-mentioned method and centrifuged at 400 g for 5 minutes to obtain a precipitate of the cells. After removing the supernatant, a Ringer's lactate solution was added thereto to resuspend the same. The number of viable cells was counted, and a Ringer's lactate solution was added so that a viable cell density became 5×10⁶ cells/mL to produce the objective material.

### Preparation of model mice and therapeutic effect

Carbon tetrachloride (CCl₄) (Wako Pure Chemical Industries, Ltd. or Sigma-Aldrich Co. LLC.) was diluted with olive oil (Wako Pure Chemical Industries, Ltd.) to prepare a solution with a concentration of 5 v/v%, and 0.5 mL/kg of CCl₄ was repeatedly administered to BALB/c mice intraperitoneally with a frequency of twice a week for 8 weeks to prepare a liver cirrhosis model. Four weeks after starting repeated administration of CCl₄, 1×10⁶ cells of the human stromal cells derived from adipose tissue were administered from tail vein. As a negative control, a group to which only a Ringer's lactate solution was administered was prepared. For evaluation, blood was collected 8 weeks after staring repeated administration of CCl₄, and biochemical examination was carried out. As a result, it was confirmed that AST and ALT showing the degree of liver cell injury showed a decreasing tendency in the cell administered group (Fig. 1).

### [Example 3] Investigation test 1 on therapeutic effect for acute hepatitis

### Preparation of administration cells

The human stromal cells derived from adipose tissue preserved as mentioned above were thawed by the above-mentioned method and centrifuged at 400 g for 5 minutes to obtain a precipitate of the cells. After removing the supernatant, the cells were suspended in PBS and seeded in a serum free medium for stromal cells (ROHTO PHARMACEUTICAL CO., LTD.) previously warmed at 37°C, cultured for 4 days, the cells were detached by the trypsin treatment and centrifuged to obtain a precipitate of the cells. After removing the supernatant, the cells were dissolved in a Ringer's lactate solution to make it 1×10⁶ cells/200 µL (Cultured MSC: G2). On the other hand, the human stromal cells derived from adipose tissue preserved as mentioned above were thawed by the above-mentioned method and centrifuged at 400 g for 5 minutes and centrifuged to obtain a precipitate of the cells. After removing the supernatant, the cells were dissolved in a Ringer's lactate solution to make it 1×10⁶ cells/200 µL (Cryopreserved MSC: G3).

### Preparation of model mice and therapeutic effect

CCl₄ was diluted with olive oil to prepare a solution with a concentration of 1.25 v/v%, and 0.0625 mL/kg of CCl₄ was intraperitoneally administered to BALB/c mice to prepare an acute hepatitis model. One day after administration of CCl₄, 1×10⁶ cells/200 µL of the human stromal cells derived from adipose tissue was administered from tail vein. As a negative control, a group to which only a Ringer's lactate solution was administered was prepared. For evaluation, blood was collected 2 days after administration of CCl₄ (one day after cell administration), and biochemical examination was carried out. As a result, it was confirmed that AST and ALT showing the degree of liver cell injury showed a decreasing tendency in the cell administered group (Fig. 2).

### [Example 4] Investigation test 2 on therapeutic effect for acute hepatitis

### Preparation of administration cells

The human stromal cells derived from adipose tissue preserved as mentioned above were thawed by the above-mentioned method and centrifuged at 400 g for 5 minutes to obtain a precipitate of the cells. After removing the supernatant, the cells were suspended in PBS, and seeded in a serum free medium for stromal cells (ROHTO PHARMACEUTICAL CO., LTD.) previously warmed at 37°C, cultured for a few days, the cells were then detached by the trypsin treatment and centrifuged to obtain a precipitate of the cells. After removing the supernatant, the cells were dissolved in a Ringer's lactate solution to make it 1×10⁶ cells/200 µL.

### Preparation of model mice and therapeutic effect

CCl₄ was diluted with olive oil to prepare a solution with a concentration of 1.25 v/v%, and 0.0625 mL/kg of CCl₄ was intraperitoneally administered to BALB/c mice to prepare an acute hepatitis model. Four hours after administration of CCl₄, 1×10⁶ cells/200 µL of the human stromal cells derived from adipose tissue was administered from tail vein (G2). As a negative control, a group to which only PBS was administered was prepared (G1). On the other hand, 1×10⁶ cells/200 µL of the human stromal cells derived from adipose tissue was administered from tail vein, and 4 hours later, 0.0625 mL/kg of CCl₄ was intraperitoneally administered to prepare an acute hepatitis model (G3).

For evaluation, blood was collected 1 day after administration of CCl₄), and biochemical examination was carried out. As a result, it was confirmed that AST and ALT showing the degree of liver cell injury showed a decreasing tendency by any cell administration before and after causing hepatitis (Fig. 3).

### [Example 5] Investigation test 1 on therapeutic effect for fulminant hepatitis

### Preparation of administration cells

The human stromal cells derived from adipose tissue preserved as mentioned above were thawed by the above-mentioned method and centrifuged at 200 g for 5 minutes to obtain a precipitate of the cells. After removing the supernatant, the cells were suspended in a serum free medium for stromal cells (ROHTO PHARMACEUTICAL CO., LTD.) and centrifuged to obtain a precipitate of the cells. After removing the supernatant, the cells were dissolved in a Ringer's lactate solution to make it 1×10⁶ cells/200 µL.

### Preparation of model mice and therapeutic effect

Concanavalin A type IV (Sigma-Aldrich Co. LLC.) was dissolved in PBS to prepare a solution with a concentration of 4 mg/mL, and intraperitoneally administered with 20 mL/kg to prepare a fulminant hepatitis model. After 30 minutes, the human stromal cells derived from adipose tissue were administered with 5×10⁷ cells/kg from tail vein (G2). As a negative control, a group to which only PBS was administered was prepared (G1).

For evaluation, one day after administration of concanavalin A, blood was collected and biochemical examination was carried out. As a result, it was confirmed that AST and ALT showing the degree of liver cell injury showed a decreasing tendency by administration of the cells (Fig. 4).

### [Example 6] Investigation test 2 on therapeutic effect for fulminant hepatitis

### Preparation of administration cells

The human stromal cells derived from adipose tissue preserved as mentioned above were thawed by the above-mentioned method and centrifuged at 200 g for 5 minutes to obtain a precipitate of the cells. After removing the supernatant, the cells were suspended in a serum free medium for stromal cells (ROHTO PHARMACEUTICAL CO., LTD.) and centrifuged to obtain a precipitate of the cells. After removing the supernatant, the cells were dissolved in a Ringer's lactate solution to make it 1×10⁶ cells/200 µL.

### Preparation of model mice and therapeutic effect

Concanavalin A type IV was dissolved in PBS to prepare a solution with a concentration of 4 mg/mL, and intraperitoneally administered with 20 mL/kg to prepare a fulminant hepatitis model. After 30 minutes, the human stromal cells derived from adipose tissue were administered with 1.5×10⁷ cells/kg (medium dose group, G2) and 5×10⁷ cells/kg (high dose group, G3) from tail vein. As a negative control, a group to which only a Ringer's lactate solution was administered was prepared (G1).

For evaluation, one day after administration of concanavalin A, blood was collected and biochemical examination was carried out. As a result, it was confirmed that AST and ALT showing the degree of liver cell injury showed a decreasing tendency by administration of the cells (Fig. 5).

### [Example 7] Investigation test 3 on therapeutic effect for acute hepatitis

### Preparation of administration cells

The human stromal cells derived from adipose tissue preserved as mentioned above were thawed by the above-mentioned method and STEM-CELLBANKER was added thereto to make it 1×10⁶ cells/200 µL.

### Preparation of model mice and therapeutic effect

CCl₄ was diluted with olive oil to prepare a solution with a concentration of 1.25 v/v%, and 0.0625 mL/kg of CCl₄ was intraperitoneally administered to BALB/c mice to prepare an acute hepatitis model. One day after administration of CCl₄, 1×10⁶ cells/200 µL of the human stromal cells derived from adipose tissue was administered from tail vein (G3). As a comparison subject, groups to which 200 µL to 800 µL of STEM-CELLBANKER were administered were prepared (G2). On the other hand, 200 µL to 800 µL of STEM-CELLBANKER were administered to a healthy model to which only olive oil was administered to BALB/c mice (G1).

For evaluation, 2 days after administration of CCl₄ (one day after cell administration), blood was collected and biochemical examination was carried out. and biochemical examination was carried out. As a result, it was confirmed that STEM-CELLBANKER itself did not induce hepatitis, and did not have a therapeutic effect of hepatitis. Also, it was confirmed that AST and ALT showing the degree of liver cell injury showed a decreasing tendency even when the cells were administered together with STEM-CELLBANKER (Fig. 6).

### [Example 8] Investigation test 2 on therapeutic effect for liver cirrhosis

### Preparation of administration cells

The human stromal cells derived from adipose tissue (hADSC, Lonza) which had been cryopreserved were thawed by the above-mentioned method and centrifuged at 200 g for 5 minutes to obtain a precipitate of the cells. After removing the supernatant, the cells were suspended in PBS, seeded in a serum free medium for stromal cells (ROHTO PHARMACEUTICAL CO., LTD.) previously warmed at 37°C and cultured for 4 days. The cells thus obtained were detached by the accutase treatment and centrifuged to obtain a precipitate of the cells. After removing the supernatant, the cells were dissolved in a Ringer's lactate solution to make it 5.4×10⁶ cells/mL.

### Preparation of model mice and therapeutic effect

CCl₄ was diluted with olive oil to prepare solutions with concentrations of 10 v/v% and 25 v/v%, and by appropriately adjusting the administration dose, a dose of 1 mL/kg of CCl₄ was repeatedly administered to BALB/c mice intraperitoneally with a frequency of twice a week for 8 weeks to prepare a liver cirrhosis model. Four weeks after starting repeated administration of CCl₄, 1×10⁶ cells of the human stromal cells derived from adipose tissue were administered from tail vein (10%+MSC group and 25%+MSC group). As a negative control, groups to which no stem cells derived from human adipose was administered (10% group and 25% group) were prepared. In addition, normal animals (Normal group) without administering CCl₄ were prepared.

For evaluation, 8 weeks after starting repeated administration of CCl₄, the liver was excised, and the degree of hepatic fibrosis was evaluated by a hepatic fibrosis scoring due to observation of Masson's trichrome stained specimen and measurement of the content of hydroxyproline in the liver tissue. As a result, it was confirmed that hepatic fibrosis was suppressed in the group administered with the cells regardless of the concentration of CCl₄ at the time of induction of hepatic fibrosis (Fig. 7A and B).

### [Example 9] Investigation test 3 on therapeutic effect for liver cirrhosis

### Preparation of administration cells

The human stromal cells derived from adipose tissue preserved as mentioned above were thawed by the above-mentioned method and centrifuged at 200 g for 5 minutes to obtain a precipitate of the cells. After removing the supernatant, the cells were suspended in a Ringer's lactate solution to make it 5×10⁶ cells/mL.

### Preparation of model mice and therapeutic effect

CCl₄ was diluted with olive oil to prepare solutions with concentrations of 50 v/v% and 75 v/v%, and by appropriately adjusting the administration dose, a dose of 1 mL/kg of CCl₄ was repeatedly administered to BALB/c mice intraperitoneally with a frequency of twice a week for 8 weeks to prepare a liver cirrhosis model. Four weeks after starting repeated administration of CCl₄, 1×10⁶ cells of the human stromal cells derived from adipose tissue were administered from tail vein (CCl₄+MSC). As a negative control, a group to which CCl₄ was administered but no human stromal cells derived from adipose tissue was administered (CCl₄) was prepared. In addition, normal animals (Normal) without administering CCl₄ were prepared.

For evaluation, 8 weeks after repeated administration of CCl₄, the liver was excised, and the degree of hepatic fibrosis was evaluated by measurement of the content of hydroxyproline in the liver tissue. As a result, it was confirmed that the content of hydroxyproline in the liver showed a decreasing tendency in the cell administered group (Fig. 8). Accordingly, it was suggested the possibility that administration of the human stromal cells derived from adipose tissue could suppress fibrosis in chronic hepatitis.

### [Example 10] Investigation test on therapeutic effect for non-alcoholic steatohepatitis

### Preparation of administration cells

The human stromal cells derived from adipose tissue preserved as mentioned above were thawed by the above-mentioned method and collected in a centrifuge tube. After removing the supernatant, a Ringer's lactate solution was added to the cells so that the viable cells were adjusted to be 1.5×10⁶ cells/mL.

### Preparation of model mice and therapeutic effect

By feeding choline-deficient amino acid-defined diet (CDAA) to KK-A^{y} mice for 9 weeks, non-alcoholic steatohepatitis model mice (NASH) were prepared. From the 8th day of feed ingestion, 3×10⁵ cells of the human stromal cells derived from adipose tissue were administered to tail vein, once every 2 weeks, in total of 4 times. As a negative control, a group of feeding normal feed and a group in which no cell was administered to each non-alcoholic steatohepatitis model mice were prepared.

On the next day after the end of the feeding period, the liver was excised, and the degree of hepatic fibrosis was evaluated by measurement of the content of hydroxyproline in the liver tissue. As a result, lowering in the content of hydroxyproline in the liver tissue was confirmed in the cell administered group (Fig. 9). Accordingly, it was suggested the possibility that administration of the human stromal cells derived from adipose tissue could suppress fibrosis in non-alcoholic steatohepatitis.

### [Example 11] Investigation test 4 on therapeutic effect for liver cirrhosis

### Preparation of administration cells

Two kinds of the stromal cells derived from adipose tissue (TRB02 or TRB03) preserved as mentioned above were thawed by the above-mentioned method and collected in centrifugal tubes. After centrifugation, the supernatant was removed, and 2 mL of a Ringer's lactate solution was added to the cells. The Ringer's lactate solution was appropriately added to adjust the viable cells to be 5×10⁶ cells/mL.

### Preparation of model mice and therapeutic effect

CCl₄ was diluted with olive oil to prepare a solution with a concentration of 10 v/v%, and it was repeatedly administered to BALB/c mice intraperitoneally with a volume of 10 mL/kg and with a frequency of twice a week for 8 weeks to prepare a liver cirrhosis model. Four weeks after repeated administration of CCl₄, 1×10⁶ cells of the human stromal cells derived from adipose tissue (TRB02 or TRB03) were administered from tail vein (single administration). Also, 0, 2, 4 and 6 weeks after starting repeated administration of CCl₄, 1×10⁶ cells of the human stromal cells derived from adipose tissue were administered from tail vein (administered biweekly 4 times in total). As a negative control, a group to which no CCl₄ was administered and a group to which CCl₄ was administered but no human stromal cells derived from adipose tissue was administered were prepared.

For evaluation, 8 weeks after repeated administration of CCl₄, the liver was excised, and the degree of hepatic fibrosis was evaluated by measurement of the content of hydroxyproline in the liver tissue. As a result, it was confirmed that the content of hydroxyproline in the liver tissue was significantly lowered in the cell administered group (Fig. 10). Accordingly, it was suggested the possibility that administration of the human stromal cells derived from adipose tissue could suppress fibrosis in liver cirrhosis even with a single administration.

### [Example 12]

### Evaluation of anti-inflammatory effect

2×10⁵ cells of THP-1 cells (RIKEN BRC) were seeded in a 12-well plate and cultured in the presence of 100 nM Phorbol 12-Myristate 13-Acetate (Wako Pure Chemical Industries, Ltd.) for 72 hours to induce differentiation into macrophages. After induction of differentiation of macrophages, activation was induced by adding 1 µg/ml of LPS (InvivoGen) and co-culture with human stromal cells derived from adipose tissue (hADSC, Lonza, number of passage: 4) was started. Co-culture was carried out using Falcon cell culture insert (Corning), and the human stromal cells derived from adipose tissue were seeded on the upper chamber. 24 hours after LPS stimulation, THP-1 macrophages were recovered from the lower chamber, mRNA expression of inflammatory cytokines (IL6, CCL2, IL1β and TNF) was measured by quantitative PCR (Fig. 11).

As a result, it was confirmed that induction of inflammatory cytokine expression by LPS stimulation is suppressed by co-culture with human stromal cells derived from adipose tissue. Accordingly, it was suggested that the human stromal cells derived from adipose tissue have anti-inflammatory action.

## Claims

1. A therapeutic agent for use in the treatment of liver diseases which comprises stromal cells derived from adipose tissue subjected to both cryopreservation and thawing at least twice, wherein the treatment comprises administering the cells immediately after said thawing.

2. The therapeutic agent for use in the treatment of liver disease according to Claim 1, which is for treating hepatitis.

3. The therapeutic agent for use in the treatment of liver disease according to Claim 1, which is for treatment of hepatic cirrhosis.

4. The therapeutic agent for use in the treatment of liver disease according to any one of Claims 1 to 3, which is for suppressing liver fibrosis.

5. The therapeutic agent for use in the treatment of liver disease according to any one of Claims 1 to 4, wherein the stromal cells derived from adipose tissue are allogeneic to a subject.

6. The therapeutic agent for use in the treatment of liver disease according to any one of Claims 1 to 5, wherein the stromal cells derived from adipose tissue are human stromal cells derived from adipose tissue.

7. The therapeutic agent for use in the treatment of liver disease according to any one of Claims 1 to 6, wherein an action of subjecting to both cryopreservation and thawing at least twice contains the following steps of;
(1) a step of cryopreserving and thawing stromal cells derived from adipose tissue,
(2) a step of culturing the thawed cells, and
(3) a step of cryopreserving and thawing the cultured cells.

8. The therapeutic agent for use in the treatment of liver disease according to any one of Claims 1 to 7, wherein the stromal cells derived from adipose tissue are stromal cells derived from adipose tissue at passage 4 or less.

9. The therapeutic agent for use in the treatment of liver disease according to any one of Claims 1 to 8, wherein the cryopreservation is carried out in a gas phase on a liquid nitrogen.

10. The therapeutic agent for use in the treatment of liver disease according to any one of Claims 1 to 9, wherein the cryopreservation is carried out by using a solution containing DMSO (Dimethyl sulfoxide).

11. A method for producing a therapeutic agent for use in the treatment of liver diseases containing stromal cells derived from adipose tissue which comprises
(i) a step of culturing stromal cells separated from collected adipose tissue,
(ii) a step of subjecting to both cryopreservation and thawing the stromal cells derived from adipose tissue obtained in the step (i),
(iii) a step of subjecting to both cryopreservation and thawing the thawed stromal cells derived from adipose tissue, and
(iv) a step of mixing the thawed stromal cells derived from adipose tissue in the step (iii) with an infusion preparation.

12. The producing method according to Claim 11, wherein the cryopreservation is carried out by using a cryopreservation solution, and the step (iv) is a step of mixing a cryopreservation solution containing the thawed stromal cells derived from adipose tissue with an infusion preparation.

13. The producing method according to Claim 11 or 12, wherein the stromal cells derived from adipose tissue are human stromal cells derived from adipose tissue.

14. The producing method according to any one of Claims 11 to 13, wherein the culturing in the step (i) is carried out at least one passage.

15. The producing method according to any one of Claims 11 to 14, wherein a step of culturing the thawed stromal cells derived from adipose tissue is further contained in the step (ii).

## Patentansprüche

1. Therapeutikum zur Verwendung bei der Behandlung von Lebererkrankungen, das aus adipösem Gewebe gewonnene Stromazellen umfasst, die zumindest zweimal jeweils einer Kryokonservierung und einem Auftauen unterzogen wurden, wobei die Behandlung das Verabreichen der Zellen unmittelbar nach dem Auftauen umfasst.

2. Therapeutikum zur Verwendung bei der Behandlung von Lebererkrankungen nach Anspruch 1, das zur Behandlung von Hepatitis dient.

3. Therapeutikum zur Verwendung bei der Behandlung von Lebererkrankungen nach Anspruch 1, das zur Behandlung von Leberzirrhose dient.

4. Therapeutikum zur Verwendung bei der Behandlung von Lebererkrankungen nach einem der Ansprüche 1 bis 3, das zum Supprimieren von Leberfibrose dient.

5. Therapeutikum zur Verwendung bei der Behandlung von Lebererkrankungen nach einem der Ansprüche 1 bis 4, wobei die aus adipösem Gewebe gewonnenen Stromazellen für ein Individuum allogen sind.

6. Therapeutikum zur Verwendung bei der Behandlung von Lebererkrankungen nach einem der Ansprüche 1 bis 5, wobei die aus adipösem Gewebe gewonnenen Stromazellen aus adipösem Gewebe gewonnene menschliche Stromazellen sind.

7. Therapeutikum zur Verwendung bei der Behandlung von Lebererkrankungen nach einem der Ansprüche 1 bis 6, wobei der Vorgang des zumindest zweimaligen Unterziehens jeweils der Kryokonservierung und des Auftauens die folgenden Schritte enthält;
(1) einen Schritt des Kryokonservierens und Auftauens von Stromazellen, die aus adipösem Gewebe stammen,
(2) einen Schritt des Kultivierens der aufgetauten Zellen und
(3) einen Schritt des Kryokonservierens und Auftauens der kultivierten Zellen.

8. Therapeutikum zur Verwendung bei der Behandlung von Lebererkrankungen nach einem der Ansprüche 1 bis 7, wobei die aus adipösem Gewebe gewonnenen Stromazellen Stromazellen sind, die aus adipösem Gewebe nach der 4. Passage oder weniger gewonnen wurden.

9. Therapeutikum zur Verwendung bei der Behandlung von Lebererkrankungen nach einem der Ansprüche 1 bis 8, wobei die Kryokonservierung in einer Gasphase auf flüssigem Stickstoff durchgeführt wird.

10. Therapeutikum zur Verwendung bei der Behandlung von Lebererkrankungen nach einem der Ansprüche 1 bis 9, wobei die Kryokonservierung unter Verwendung einer DMSO (Dimethylsulfoxid) enthaltenden Lösung durchgeführt wird.

11. Verfahren zur Herstellung eines Therapeutikums zur Verwendung bei der Behandlung von Lebererkrankungen, das aus adipösem Gewebe gewonnene Stromazellen umfasst, das Folgendes umfasst:
(i) einen Schritt des Kultivierens von Stromazellen, die von abgenommenem adipösem Gewebe getrennt wurden,
(ii) einen Schritt des Unterziehens der aus adipösem Gewebe gewonnenen Stromazellen, die in Schritt (i) erhalten wurden, sowohl gegenüber einer Kryokonservierung als auch einem Auftauen,
(iii) einen Schritt des Unterziehens der aufgetauten, aus adipösem Gewebe gewonnenen Stromazellen sowohl gegenüber einer Kryokonservierung als auch einem Auftauen und
(iv) einen Schritt des Mischens der aufgetauten, aus adipösem Gewebe gewonnenen Stromazellen aus Schritt (iii) mit einem Infusionspräparat.

12. Herstellungsverfahren nach Anspruch 11, wobei die Kryokonservierung unter Verwendung einer Kryokonservierungslösung durchgeführt wird und Schritt (iv) einen Schritt des Mischens einer Kryokonservierungslösung, die die aufgetauten, aus adipösem Gewebe gewonnenen Stromazellen enthält, mit einem Infusionspräparat darstellt.

13. Herstellungsverfahren nach Anspruch 11 oder 12, wobei die aus adipösem Gewebe gewonnenen Stromazellen aus adipösem Gewebe gewonnene menschliche Stromazellen sind.

14. Herstellungsverfahren nach einem der Ansprüche 11 bis 13, wobei das Kultivieren in Schritt (i) mit zumindest einer Passage durchgeführt wird.

15. Herstellungsverfahren nach einem der Ansprüche 11 bis 14, wobei in Schritt (ii) weiters ein Schritt des Kultivierens der aufgetauten, aus adipösem Gewebe gewonnenen Stromazellen enthalten ist.

## Revendications

1. Agent thérapeutique à utiliser dans le traitement de maladies du foie qui comprend des cellules stromales dérivées de tissu adipeux soumises à la fois à une cryoconservation et à une décongélation au moins deux fois, dans lequel le traitement comprend l'administration des cellules immédiatement après ladite décongélation.

2. Agent thérapeutique à utiliser dans le traitement d'une maladie du foie selon la revendication 1, qui est destiné au traitement de l'hépatite.

3. Agent thérapeutique à utiliser dans le traitement d'une maladie du foie selon la revendication 1, qui est destiné au traitement de la cirrhose hépatique.

4. Agent thérapeutique destiné à être utilisé dans le traitement d'une maladie du foie selon l'une quelconque des revendications 1 à 3, qui est destiné à supprimer une fibrose du foie.

5. Agent thérapeutique à utiliser dans le traitement d'une maladie du foie selon l'une quelconque des revendications 1 à 4, dans lequel les cellules stromales dérivées d'un tissu adipeux sont allogéniques par rapport à un sujet.

6. Agent thérapeutique destiné à être utilisé dans le traitement d'une maladie du foie selon l'une quelconque des revendications 1 à 5, dans lequel les cellules stromales dérivées de tissu adipeux sont des cellules stromales humaines dérivées de tissu adipeux.

7. Agent thérapeutique à utiliser dans le traitement d'une maladie du foie selon l'une quelconque des revendications 1 à 6, dans lequel une action consistant à soumettre à la fois à une cryoconservation et à une décongélation au moins deux fois contient les étapes suivantes :
(1) une étape de cryoconservation et de décongélation de cellules stromales dérivées de tissu adipeux,
(2) une étape de culture des cellules décongelées, et
(3) une étape de cryoconservation et de décongélation des cellules cultivées.

8. Agent thérapeutique à utiliser dans le traitement d'une maladie du foie selon l'une quelconque des revendications 1 à 7, dans lequel les cellules stromales dérivées de tissu adipeux sont des cellules stromales dérivées de tissu adipeux inférieures ou égales au niveau du passage 4.

9. Agent thérapeutique destiné à être utilisé dans le traitement d'une maladie du foie selon l'une quelconque des revendications 1 à 8, dans lequel la cryoconservation est effectuée en phase gazeuse sur un azote liquide.

10. Agent thérapeutique à utiliser dans le traitement d'une maladie du foie selon l'une quelconque des revendications 1 à 9, dans lequel la cryoconservation est effectuée en utilisant une solution contenant du DMSO (diméthylsulfoxyde).

11. Procédé de production d'un agent thérapeutique à utiliser dans le traitement de maladies du foie contenant des cellules stromales dérivées du tissu adipeux, qui comprend
(i) une étape de culture de cellules stromales séparées du tissu adipeux collecté,
(ii) une étape consistant à soumettre à la fois à une cryoconservation et à une décongélation les cellules stromales dérivées de tissu adipeux obtenues à l'étape (i),
(iii) une étape consistant à soumettre à la fois à une cryoconservation et à une décongélation les cellules stromales décongelées dérivées du tissu adipeux, et
(iv) une étape de mélange des cellules stromales décongelées dérivées de tissu adipeux dans l'étape (iii) avec une préparation de perfusion.

12. Procédé de production selon la revendication 11, dans lequel la cryoconservation est effectuée en utilisant une solution de cryoconservation, et l'étape (iv) est une étape consistant à mélanger une solution de cryoconservation contenant les cellules stromales décongelées dérivées de tissu adipeux avec une préparation de perfusion.

13. Procédé de production selon la revendication 11 ou 12, dans lequel les cellules stromales dérivées de tissu adipeux sont des cellules stromales humaines dérivées de tissu adipeux.

14. Procédé de production selon l'une quelconque des revendications 11 à 13, dans lequel la culture dans l'étape (i) est effectuée au niveau d'au moins un passage.

15. Procédé de production selon l'une quelconque des revendications 11 à 14, dans lequel une étape de culture des cellules stromales décongelées dérivées du tissu adipeux est en outre contenue dans l'étape (ii).
